(19) Europäisches Patentamt
European Patent Office
Office européen des brevets

(11) **EP 4 074 247 B1**

(12) **EUROPEAN PATENT SPECIFICATION**

(45) Date of publication and mention
of the grant of the patent:
**26.11.2025 Bulletin 2025/48**

(21) Application number: **20912646.5**

(22) Date of filing: **10.12.2020**

(51) International Patent Classification (IPC):
***A61B 5/00*** (2006.01)

(52) Cooperative Patent Classification (CPC):
**A61B 5/0077; A61B 5/004; A61B 5/02416;
A61B 5/7267; H04N 23/611; H04N 23/90;**
A61B 5/44

(86) International application number:
**PCT/CN2020/135090**

(87) International publication number:
**WO 2021/139471 (15.07.2021 Gazette 2021/28)**

(54) **HEALTH STATUS TEST METHOD AND DEVICE, AND COMPUTER STORAGE MEDIUM**

VERFAHREN UND VORRICHTUNG ZUR PRÜFUNG DES GESUNDHEITSZUSTANDS UND
COMPUTERSPEICHERMEDIUM

PROCÉDÉ ET DISPOSITIF DE TEST D'ÉTAT DE SANTÉ ET SUPPORT DE STOCKAGE
INFORMATIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **06.01.2020 CN 202010009254**

(43) Date of publication of application:
**19.10.2022 Bulletin 2022/42**

(73) Proprietor: **Huawei Technologies Co., Ltd.
Shenzhen, Guangdong 518129 (CN)**

(72) Inventors:
• **CUI, Xiaoying
Shenzhen, Guangdong 518129 (CN)**
• **QIN, Chuan
Shenzhen, Guangdong 518129 (CN)**

• **WANG, Xiaomei
Shenzhen, Guangdong 518129 (CN)**

(74) Representative: **Maiwald GmbH
Elisenhof
Elisenstraße 3
80335 München (DE)**

(56) References cited:
**EP-A1- 3 725 217          WO-A1-2019/172642
AU-A1- 2015 201 759    CN-A- 108 335 749
CN-A- 108 399 364       CN-A- 108 509 905
CN-A- 108 734 676       CN-A- 109 363 634
CN-A- 110 049 240       CN-A- 110 134 316
CN-A- 111 166 290       CN-B- 106 210 532
US-A1- 2013 108 123**

## Description

### TECHNICAL FIELD

The present invention relates to the field of image analysis technologies, and specifically, to a health status detection method and device, and a computer storage medium.

### BACKGROUND

CN109363634A relates to a method, device, mobile phone, computer device and storage medium for evaluating skin, and belongs to the technical field of evaluation. It is described that the method comprises the following steps of: sending an illumination instruction to an illumination unit; wherein the illumination instruction is used for triggering the illumination unit to emit specific skin test light; the skin test light is used to irradiate the skin of a subject; acquiring a skin image acquired by a shooting device; wherein the skin image includes an image of the skin of the subject when irradiated by the skin test light; evaluating a skin state of the subject based on the skin image.

CN108509905A describes a health status evaluation method and device, electronic equipment and a computer-readable storage medium. It is described that the health status evaluation method of an entrance/exit includes: acquiring a face image of a user, wherein the step includes acquiring the face image of the user at a fixed time period, and/or selecting the face image acquired in a fixed scene; recognizing facial features of the acquired face image; and evaluating health status of the user according to the recognized facial features of the face image.

As the pace of life accelerates, young people are busy at work and may have no time to pay attention to their own health status. In the past, to detect a health status, people need to go to a hospital to queue, or purchase some diagnostic devices such as a sphygmomanometer. However, such a detection process is usually long and cumbersome. Therefore, many applications (Application, app for short) emerge in the market, for example, Meitu makeup, AiSkinCare, Heart rate monitor, and Body mass index (Body Mass Index, BMI for short) test. However, when these health applications detect a health status, a user needs to manually start the health applications and obtain a user health status by photographing at a fixed location or photographing assisted by another person.

### SUMMARY

In view of this, the present invention provides a health status detection method and device, and a computer storage medium, to automatically perform health status detection, so as to implement senseless health status detection.

The invention and its scope of protection is defined by the appended independent claims. Embodiments of the invention are defined by the appended dependent claims. The following aspects and implementations of the disclosure provide examples of combinations of technical subject matters.

According to a first aspect, an embodiment of the present invention provides a health status detection method. The method is applied to an electronic device having a display and a camera, and the camera includes a first camera or the camera includes a first camera and a second camera. The method includes:

when it is detected that the display is in a working state, controlling the first camera to collect an environmental image;
obtaining detection parameters from the environmental image;
determining whether the detection parameters meet detection conditions corresponding to the detection parameters;
if it is determined that the detection parameters meet the detection conditions corresponding to the detection parameters, controlling the second camera or the first camera to collect a face image; and
generating health status data based on the face image.

In a possible implementation, the generating at least one piece of health status data based on the face image includes:

inputting the face image into a pre-established neural network model, and outputting the at least one piece of health status data. In a possible implementation, the at least one piece of health status data includes a skin status, a heart rate, blood pressure, and body fat.

In a possible implementation, the detection parameters include an environmental luminance, a face image range, and duration in which the face remains static; and the determining whether the detection parameters meet detection conditions corresponding to the detection parameters includes:

determining whether the face image range is within a preset position range, whether the environmental luminance is greater than or equal to a first preset threshold, and whether the duration in which the face remains static is greater than or equal to a second preset threshold.

In a possible implementation, after the generating health status data based on the face image, the method further includes:

determining whether the health status data is within a preset health data range, and generating a health status evaluation result based on a determining result.

In a possible implementation, the first camera includes a low-power camera.

According to a second aspect, an embodiment of the present invention provides an electronic device, including: a detection unit, configured to detect whether a display is in a working state; and a processing unit, configured to: when the detection unit detects that the display is in the working state, control the first camera to collect an environmental image. The processing unit is further configured to obtain detection parameters from the environmental image. The processing unit is further configured to determine whether the detection parameters meet detection conditions corresponding to the detection parameters; and if the detection unit determines that the detection parameters meet the detection conditions corresponding to the detection parameters, control the second camera or the first camera to collect a face image. The processing unit is further configured to generate at least one piece of health status data based on the face image.

In a possible implementation, the processing unit is configured to input the face image into a pre-established neural network model, and output the at least one piece of health status data. In a possible implementation, the health status data includes at least one of a skin status, a heart rate, blood pressure, and body fat.

In a possible implementation, the detection parameters include an environmental luminance, a face image range, and duration in which a face remains static.

In a possible implementation, the processing unit is configured to determine whether the face image range is within a preset position range, whether the environmental luminance is greater than or equal to a first preset threshold, and whether the duration in which the face remains static is greater than or equal to a second preset threshold.

In a possible implementation, the processing unit is configured to determine whether the health status data is within a preset health data range, and generate a health status evaluation result based on a determining result.

In a possible implementation, the first camera includes a low-power camera.

According to a third aspect, an embodiment of the present invention provides an electronic device, including a display, a first camera, a second camera, one or more processors, a memory, a plurality of applications, and one or more computer programs, where the one or more computer programs are stored in the memory. The one or more computer programs include instructions, and when the instructions are executed by the device, the device is enabled to perform the information display method in any possible implementation of any one of the foregoing aspects.

According to a fourth aspect, an embodiment of the present invention provides a computer storage medium. The computer storage medium stores program code to be executed by a device. The program code includes instructions for performing the method in any one of the first aspect or the possible implementations of the first aspect.

In the technical solution provided in embodiments of the present invention, the first camera is controlled to collect the environmental image, and the detection parameters are obtained from the environmental image. If it is determined that the detection parameters meet the detection conditions corresponding to the detection parameters, the second camera or the first camera is controlled to collect the face image, and health status data is generated based on the face image. In embodiments of the present invention, the first camera collects the environmental image to determine the detection conditions corresponding to the detection parameters, and the second camera or the first camera is controlled to collect the face image to generate the health status data. A user does not need to manually perform health status detection, but health status detection can be automatically performed, to implement senseless health status detection.

## BRIEF DESCRIPTION OF DRAWINGS

FIG. 1 is a schematic diagram of a structure of a system architecture according to an embodiment of the present invention;

FIG. 2 is a schematic diagram of a structure of a convolutional neural network according to an embodiment of the present invention;

FIG. 3 is a schematic diagram of a structure of an electronic device having a display and a camera according to an embodiment of the present invention;

FIG. 4 is a locally enlarged schematic diagram of FIG. 3;

FIG. 5 is a schematic diagram of image collection ranges of a first camera and a second camera according to an embodiment of the present invention;

FIG. 6 is a schematic diagram of a structure of another system architecture according to an embodiment of the present invention;

FIG. 7 is a schematic diagram of a structure of another electronic device having a display and a camera according to an embodiment of the present invention;

FIG. 8 is a flowchart of a health status detection method according to an embodiment of the present invention;

FIG. 9 is a schematic diagram of identifying a face image range according to an embodiment of the present invention;

FIG. 10 is a schematic diagram of a structure of an electronic device according to an embodiment of the present invention; and

FIG. 11 is a schematic diagram of a structure of another electronic device according to an embodiment of the present invention.

## DESCRIPTION OF EMBODIMENTS

To make the technical solutions in the present invention more comprehensible, the following describes embodiments of the present invention in detail with reference to the accompanying drawings.

It should be clear that the described embodiments are merely a part rather than all of embodiments of the present invention. All other embodiments obtained by a person of ordinary skill in the art based on embodiments of the present invention without creative efforts shall fall within the protection scope of the present invention.

The terms used in embodiments of the present invention are merely for the purpose of illustrating specific embodiments, and are not intended to limit the present invention. The terms "a", "this", and "the" of singular forms used in embodiments and the appended claims of the present invention are also intended to include plural forms, unless otherwise specified in the context clearly.

It should be understood that the term "and/or" used in this specification describes only an association relationship between associated objects and represents that three relationships may exist. For example, A and/or B may represent the following three cases: Only A exists, both A and B exist, and only B exists. In addition, the character "/" in this specification indicates an "or" relationship between the associated objects.

A health status detection method provided in embodiments of the present invention can be used in a scenario in which a display of an electronic device is in a working state. The following briefly describes the scenario in which the display is in the working state.

The scenario in which the display is in the working state is described as follows:

When the display is in a screen-on state, the display is in the working state. For example, in a scenario in which the user views a document, watches a video, or takes a picture by using the electronic device, or the user performs social chatting by using the electronic device, the display is in the screen-on state, and it indicates that the display is in the working state. Each of the foregoing scenarios in which the display is in the working state is the scenario in which the display is in the working state.

In the scenario in which the display is in the working state, the technical solution for detecting a health status provided in embodiments of the present invention is used. A first camera collects an environmental image to determine detection conditions corresponding to detection parameters, so that a second camera or the first camera is controlled to collect a face image to generate health status data. This reduces complexity of a health status detection process. In addition, in the health status detection process, the user does not need to manually open a health application and photograph an image to perform health detection, but implements "senseless" health status detection when the user normally uses the electronic device. This improves health detection efficiency. In this way, the user can obtain health status information in real time when a health status detection condition is met, and the user can learn about the health status information of the user in real time and monitor a physical status, to improve quality of a physical health status of the user.

Application of neural network is described in embodiments of the present invention. Therefore, for ease of understanding, the following first describes possible related terms and related concepts such as a neural network in embodiments of the present invention.

(1) Neural network

The neural network may include a neuron. The neuron may be an operation unit that uses $x_s$ and an intercept of b as an input, and an output of the operation unit may be as follows:

$$h_{W,b}\left(x\right) = f\left(W^T x\right) = f\left(\sum\nolimits_{s=1}^{n} W_s x_s + b\right) \qquad (1\text{-}1)$$

Herein, s = 1, 2, ..., or n, n is a natural number greater than 1, $W_s$ is a weight of $x_s$, b is a bias of the neuron, and f is an activation function (activation functions) of the neuron, and the activation function is used for introducing a non-linear characteristic into the neural network, to convert an input signal in the neuron into an output signal. The output signal of the activation function may be used as an input of a next convolution layer. The activation function may be a sigmoid function. The neural network is a network formed by joining many single neurons together. To be specific, an output of a neuron may be an input of another neuron. An input to each neuron may be connected to a local receptive field of a previous layer to extract a feature of the local receptive field. The local receptive field may be a region including several neurons.

(2) Deep neural network

The deep neural network (deep neural network, DNN for short) is also referred to as a multi-layer neural network, and may be understood as a neural network having many hidden layers. There is no special measurement criterion for the "many" herein. Based on locations of different layers in the DNN, a neural network in the DNN may be divided into three types: an input layer, a hidden layer, and an output layer. Generally, a first layer is the input layer, a last layer is the output layer, and a middle layer is the hidden layer. For example, in a fully connected neural network, layers are fully connected. In other words, any neuron at the $i^{th}$ layer needs to be connected to any neuron at the $(i+1)^{th}$ layer. Although the DNN seems complex, it is not complex for each layer. Simply speaking, the DNN is the following linear relationship expression:

$$\vec{y} = \alpha(W\vec{x} + \vec{b})$$

Herein, $\vec{x}$ is an input vector, $\vec{y}$ is an output vector, $\vec{b}$ is an offset vector, $W$ is a weight matrix (also referred to as a coefficient), and $\alpha()$ is an activation function. In each layer, only such a simple operation is performed on the input vector to obtain the output vector. Because there are many layers in the DNN, there are also many coefficients $W$ and bias vectors $\vec{b}$. Definitions of these parameters in the DNN are as follows. The coefficient W is used as an example. It is assumed that in a DNN having three layers, a linear coefficient from the fourth neuron at the second layer to the second neuron at the third layer is defined as $W_{24}^{3}$. A superscript 3 indicates an ordinal number of a layer at which the coefficient $W$ is located, and a subscript corresponds to an index 2 of the third layer for output and an index 4 of the second layer for input. In conclusion, a coefficient from the $k^{th}$ neuron at the $(L-1)^{th}$ layer to the $j^{th}$ neuron at the $L^{th}$ layer is defined as $W_{jk}^{L}$. It should be noted that the input layer has no parameter $W$. In the deep neural network, more hidden layers allow the network to better describe a complex case in the real world. Theoretically, a model with more parameters has higher complexity and a larger "capacity". It indicates that the model can complete a more complex learning task. Training the deep neural network is a process of learning a weight matrix, and a final objective of the training is to obtain a weight matrix of all layers of a trained deep neural network (a weight matrix formed by vectors W at many layers).

(3) Convolutional neural network

The convolutional neural network (convolutional neural network, CNN for short) is a deep neural network with a convolutional structure. The convolutional neural network includes a feature extractor consisting of a convolutional layer and a sub sampling layer. The feature extractor may be considered as a filter. A convolution process may be considered as performing convolution by using a trainable filter and an input image or a convolution feature map (feature map). The convolutional layer is a neuron layer, in a convolutional neural network, that performs convolution processing on an input signal. In the convolutional layer of the convolutional neural network, one neuron may be connected to only a part of neurons in a neighboring layer. A convolutional layer generally includes several feature planes, and each feature plane may include some neurons arranged in a rectangle. Neurons of a same feature plane share a weight, and the shared weight herein is a convolution kernel. Sharing a weight may be understood as that a manner of extracting image information is unrelated to a position. The principles implied herein are that statistical information of a part of an image is the same as that of another part. To be specific, image information that is learned in a part can also be used in another part. Therefore, same learned image information can be used for all locations in the image. At a same convolutional layer, a plurality of convolution kernels may be used to extract different image information. Usually, a larger quantity of convolution kernels indicates richer image information reflected by a convolution operation.

The convolution kernel may be initialized in a form of a matrix of a random size. In a training process of the convolutional neural network, an appropriate weight may be obtained for the convolution kernel through learning. In addition, sharing the weight is advantageous because connections between layers of the convolutional neural network are reduced, and a risk of overfitting is reduced.

(4) Loss function

In a process of training the deep neural network, because it is expected that an output of the deep neural network is as much as possible close to a predicted value that is actually expected, a predicted value of a current network and a target value that is actually expected may be compared, and then a weight vector of each layer of the neural network is updated based on a difference between the predicted value and the target value (certainly, there is usually an initialization process before the first update, to be specific, parameters are preconfigured for all layers of the deep neural network). For example, if the predicted value of the network is large, the weight vector is adjusted to decrease the predicted value, and adjustment is continuously performed, until the deep neural network can predict the target value that is actually expected or a value that is very close to the target value that is actually expected. Therefore, "how to obtain, through comparison, a difference between the predicted value and the target value" needs to be predefined. This is a loss function (loss function) or an objective function (objective function). The loss function and the objective function are important equations that measure the difference between the predicted value and the target value. The loss function is used as an example. A higher output value (loss) of the loss function indicates a larger difference. Therefore, training of the deep neural network becomes a process of reducing the loss as much as possible.

(5) Luminance

The luminance, represented by L, is brightness of a surface, that is, a luminous flux of light reflected from the surface. In other words, the luminance is a luminous intensity of a surface light source per unit projection area in a specific direction along the specific direction. Illuminance, commonly referred to as lux (lux), indicates the luminous flux per unit area on a surface of a photographed object. 1 lux is equivalent to 1 lumen per square meter, that is, the luminous flux vertically irradiated by a light source with luminous intensity of 1 candle at a distance of 1 meter on each square meter of the photographed subject. The illuminance is an important indicator for measuring a photographing environment.

(6) Machine learning

The machine learning (Machine Learning, ML for short) is an interdisciplinary subject involving a plurality of fields and relates to a plurality of subjects such as a probability theory, a science of statistics, an approximation theory, a convex analysis, and an algorithm complexity theory. The machine learning focuses on a study of how a computer simulates or implements learning behavior of human beings to obtain new knowledge or skills, and reorganizes an existing knowledge structure to continuously improve performance of the computer. The machine learning is a core of an artificial intelligence and a fundamental way to make computers intelligent, is widely used in various fields of artificial intelligence, and mainly uses induction and synthesis rather than deduction.

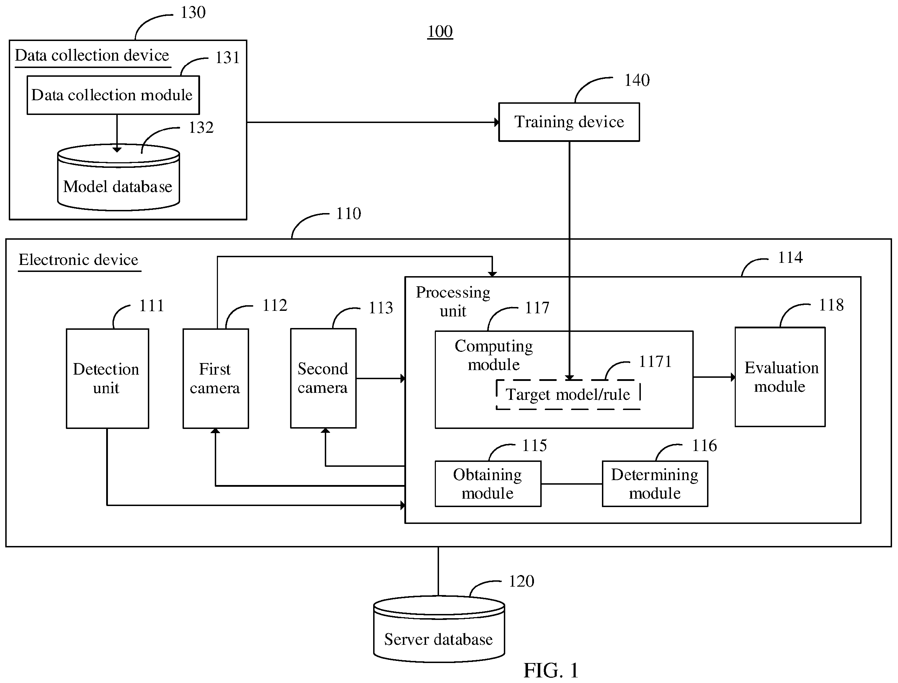

FIG. 1 is a schematic diagram of a structure of a system architecture 100 according to an embodiment of the present invention. As shown in FIG. 1, the system architecture 100 may include an electronic device 110, a data collection device 130, a training device 140, and a server database 120.

The data collection device 130 includes a data collection module 131 and a model database 132. The data collection module 131 is configured to collect training data, where the training data includes a face image. The data collection module 131 stores the training data in the model database 132. The model database 132 is configured to maintain training data.

The training device 140 obtains a target model/rule 1171 based on the training data maintained in the model database 132. The following describes how the training device 140 obtains the target model/rule 1171 based on the training data. The training device 140 processes an input face image, and outputs at least one piece of health status data, to complete training of the target model/rule 1171.

The target model/rule 1171 in embodiments of the present invention may specifically include a neural network. It should be noted that, during actual application, the training data maintained in the model database 132 is not necessarily collected by the data collection device 130, but may be received from another device. In addition, it should be noted that the training device 140 may train the target model/rule 1171 not completely based on the training data maintained in the model database 132, but may perform model training based on training data obtained from a cloud database or another database. A method for obtaining the training data is not limited in embodiments of the present invention.

The target model/rule 1171 obtained through training by the training device 140 can be applied to different systems or devices, for example, applied to an electronic device 110 shown in FIG. 1. The electronic device 110 may be a terminal such as a mobile phone terminal, a tablet computer, a notebook computer, an augmented reality (augmented reality, AR) AR/a virtual reality (virtual reality, VR) terminal, or an in-vehicle terminal; or may be a server, Cloud, or the like.

As shown in FIG. 1, the electronic device 110 includes a detection unit 111, a first camera 112, a second camera 113, and a processing unit 114.

The detection unit 111 is configured to detect whether a display is in a working state. The processing unit 114 is configured to: when the detection unit 111 detects that the display is in a working state, control the first camera 112 to collect an environmental image. The processing unit 114 is further configured to: determine whether detection parameters meet detection conditions corresponding to the detection parameters; if it is determined that the detection parameters meet the detection conditions corresponding to the detection parameters, control the second camera 113 or the first camera 112 to collect a face image; and generate health status data based on the face image. The electronic device 110 includes a health application. The processing unit 114 may start the first camera 112 over the health application, to control the first camera 112 to collect a face image. The processing unit 114 may start the second camera 113 over the health application, to control the second camera 113 to collect a face image.

Specifically, the processing unit 114 includes an obtaining module 115, a determining module 116, a calculation module 117, and an evaluation module 118. The calculation module 117 includes the target model/rule 1171.

The obtaining module 115 is configured to obtain the detection parameters from the environmental image, where the detection parameters include an environmental luminance, a face image range, and duration in which a face remains static. The determining module 116 is configured to determine whether the face image range is within a preset position range, whether the environmental luminance is greater than or equal to a first preset threshold, and whether the duration in which the face remains static is greater than or equal to a second preset threshold. When the determining module 116 determines that the face image range is within the preset position range, the environmental luminance is greater than or equal to the first preset threshold, and the duration during which the face remains static is greater than or equal to the second preset threshold, the processing unit 114 invokes the second camera 113 over the health application, to control the second camera 113 to collect a face image.

The calculation module 117 is configured to input the face image into the target model/rule 1171 and output at least one piece of health status data. The evaluation module 118 is configured to determine whether the health status data is within a preset health data range, and generate a health status evaluation result based on a determining result.

By following the foregoing procedure, the evaluation module 118 feeds back a health status evaluation result to a user. In this way, the user does not need to manually perform health status detection, but health status detection can be automatically performed, to implement senseless health status detection.

It should be noted that the training device 140 may generate a corresponding target model/rule 1171 based on different training data for different objectives or different tasks. The corresponding target model/rule 1171 may be used to implement the objectives or complete the tasks, to provide a required result for the user. For example, the health status data may be provided for the user in the present invention. When the calculation module 117 of the electronic device 110 performs a related processing process such as calculation, the electronic device 110 may invoke data, code, and the like in the server database 120 for corresponding processing, or may store data, instructions, and the like that are obtained through corresponding processing into the server database 120.

As shown in FIG. 1, a target model/rule 1171 is obtained through training by the training device 140. In embodiments of the present invention, the target model/rule 1171 may be a neural network in the present invention. To be specific, the neural network provided in embodiments of the present invention may be a convolutional neural network (convolutional neuron network, CNN), a deep convolutional neural network (deep convolutional neural networks, DCNN), or the like.

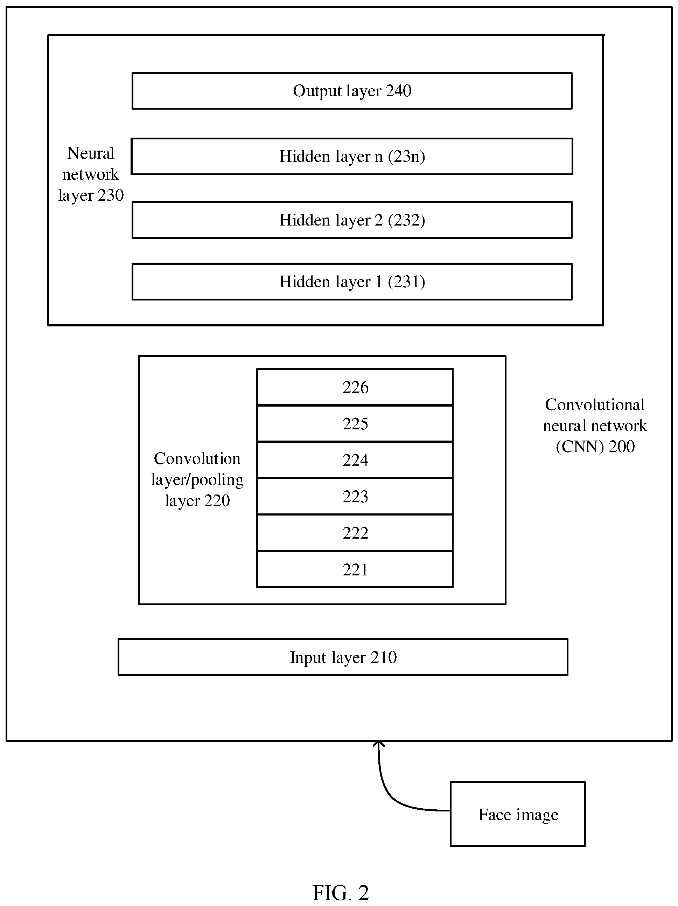

Because a convolutional neural network is a common neural network, the following mainly describes a structure of the convolutional neural network in detail with reference to FIG. 2. As described in the foregoing description of basic concepts, the convolutional neural network is a deep neural network with a convolutional structure, and is a deep learning (deep learning) architecture. The deep learning architecture is to perform multi-level learning at different abstract levels by using a machine learning algorithm. As a deep learning architecture, the convolutional neural network is a feed-forward (feed-forward) artificial neural network, and each neuron in the feed-forward artificial neural network can respond to an image input into the feed-forward artificial neural network.

As shown in FIG. 2, a convolutional neural network (CNN) 200 may include an input layer 210, a convolution layer/pooling layer 220 (the pooling layer is optional), and a neural network layer 230. The following describes related content of these layers in detail.

Convolutional layer/pooling layer 220:

Convolutional layer:

As shown in FIG. 2, the convolution layer/pooling layer 220 may include, for example, layers 221 to 226. For example, in an implementation, the layer 221 is a convolution layer, the layer 222 is a pooling layer, the layer 223 is a convolution layer, the layer 224 is a pooling layer, the layer 225 is a convolution layer, and the layer 226 is a pooling layer. In another implementation, the layers 221 and 222 are convolution layers, the layer 223 is a pooling layer, the layers 224 and 225 are convolution layers, and the layer 226 is a pooling layer. In other words, an output of a convolutional layer may be used as an input for a subsequent pooling layer, or may be used as an input for another convolutional layer, to continue to perform a convolution operation.

The following describes internal working principles of the convolutional layer by using the convolutional layer 221 as an example.

The convolutional layer 221 may include a plurality of convolution operators. The convolution operator is also referred to as a kernel. In image processing, the convolution operator functions as a filter that extracts specific information from a matrix of an input image. The convolution operator may essentially be a weight matrix, and the weight matrix is usually predefined. In a process of performing a convolution operation on an image, the weight matrix usually processes pixels at a granularity level of one pixel (or two pixels, depending on a value of a stride stride) in a horizontal direction in the input image, to extract a specific feature from the image. A size of the weight matrix is related to a size of the image.

It should be noted that a depth dimension (depth dimension) of the weight matrix is the same as a depth dimension of the input image. In a convolution operation process, the weight matrix extends to an entire depth of the input image. Therefore, a convolutional output of a single depth dimension is generated through convolution with a single weight matrix. However, in most cases, a single weight matrix is not used, but a plurality of weight matrices with a same size (rows x columns), namely, a plurality of same-type matrices, are applied. An output of each weight matrix is stacked to form a depth dimension of a convolutional image, and it may be understood that the dimension herein depends on the foregoing "plurality of".

Different weight matrices may be used to extract different features from the image. For example, one weight matrix is used to extract edge information of the image, another weight matrix is used to extract a specific color of the image, and still another weight matrix is used to blur unneeded noise in the image. Sizes of the plurality of weight matrices (rows $\times$ columns) are the same, sizes of feature maps extracted from the plurality of weight matrices with the same size are also the same, and then the plurality of extracted feature maps with the same size are combined to form an output of the convolution operation.

Weight values in these weight matrices need to be obtained through a lot of training during actual application. Each weight matrix formed by using the weight values obtained through training may be used for extracting information from an input image, to enable the convolutional neural network 200 to perform correct prediction.

When the convolutional neural network 200 has a plurality of convolutional layers, an initial convolutional layer (for example, the layer 221) usually extracts more general features, where the general features may also be referred to as low-level features. As a depth of the convolutional neural network 200 increases, a deeper convolutional layer (for example, the layer 226) extracts more complex features, such as high-level semantic features. A feature with higher semantics is more applicable to a to-be-resolved problem.

Pooling layer:

A quantity of training parameters often needs to be reduced. Therefore, a pooling layer often needs to be periodically introduced after a convolutional layer. For the layers 221 to 226 shown in 220 in FIG. 2, one convolutional layer may be followed by one pooling layer, or a plurality of convolutional layers may be followed by one or more pooling layers. During image processing, the pooling layer is only used for reducing a space size of the image. The pooling layer may include an average pooling operator and/or a maximum pooling operator, to perform sampling on the input image to obtain an image with a relatively small size. In addition, similar to that the size of the weight matrix at the convolutional layer needs to be related to the size of the image, an operator at the pooling layer also needs to be related to the size of the image. A size of a processed image output from the pooling layer may be less than a size of an image input to the pooling layer. Each pixel in the image output from the pooling layer indicates an average value or a maximum value of a corresponding sub-region of the image input to the pooling layer.

Neural network layer 230:

After processing is performed by the convolutional layer/pooling layer 220, the convolutional neural network 200 still cannot output required output information. As described above, at the convolutional layer/pooling layer 220, only a feature is extracted, and parameters resulting from an input image are reduced. However, to generate final output information (required class information or other related information), the convolutional neural network 200 needs to use the neural network layer 230 to generate an output of one required class or outputs of a quantity of a group of required classes. Therefore, the neural network layer 230 may include a plurality of hidden layers (hidden layers 231, 232, ..., and 23n shown in FIG. 2) and an output layer 240. Parameters included in the plurality of hidden layers may be obtained through pre-training based on related training data of a specific task type. For example, the task type may include image recognition, image classification, and super-resolution image reconstruction.

The layer after the plurality of hidden layers in the neural network layer 230, namely, the last layer of the entire convolutional neural network 200 is the output layer 240. The output layer 240 has a loss function similar to classification cross entropy, and is specifically configured to calculate a predicted error. Once forward propagation (propagation in a direction from the input layer 210 to the output layer 240 shown in FIG. 2 is the forward propagation) of the entire convolutional neural network 200 is complete, back propagation (propagation in a direction from the output layer 240 to the input layer 210 shown in FIG. 2 is the back propagation) starts to update a weight value and a bias of each layer mentioned above, to reduce a loss of the convolutional neural network 200, and reduce an error between an ideal result and a result output by the convolutional neural network 200 by using the output layer.

It should be noted that the convolutional neural network 200 shown in FIG. 2 is merely used as an example of a convolutional neural network. During specific application, the convolutional neural network may alternatively exist in a form of another network model.

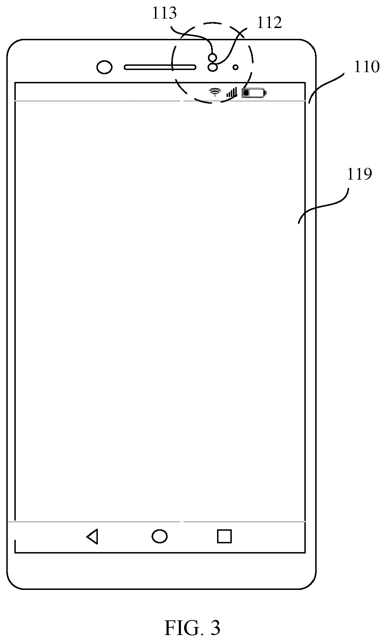

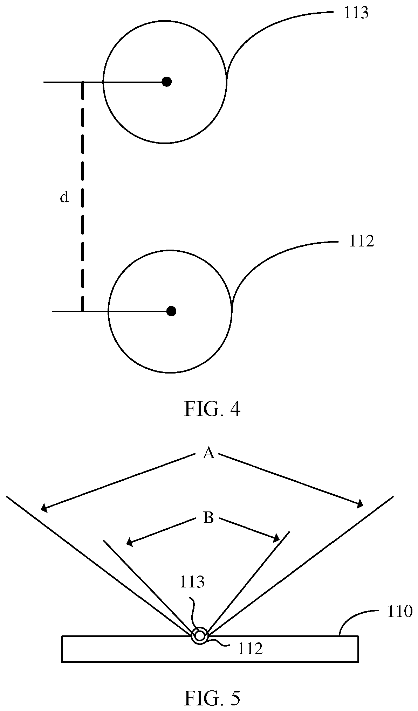

FIG. 3 is a schematic diagram of a structure of another electronic device having a display and a camera according to an embodiment of the present invention. The camera includes a first camera and a second camera. As shown in FIG. 3, the electronic device 110 includes a display 119, a first camera 112, and a second camera 113. The display 119 is configured to display a plurality of changing numbers, texts, graphic images, and the like. The first camera 112 and the second camera 113 each include a built-in camera, configured to photograph an image. A difference between the first camera 112 and the second camera 113 lies in a collected image and a range of the collected image. The first camera 112 collects an environmental image, and the second camera 113 collects a face image. In addition, both the first camera 112 and the second camera 113 are located in a front panel area of the electronic device 110. As shown in FIG. 4, a preset distance d is set between the first camera 112 and the second camera 113. For example, the preset distance d is 3 mm. As shown in FIG. 5, a range in which the first camera 112 can collect images is A, and a range in which the second camera 113 can collect images is B. The image range A is greater than the image range B.

In embodiments of the present invention, an image collected by the first camera is an environmental image, and is for obtaining detection parameters. The image collection range of the first camera is set to be greater than the image collection range of the second camera. This helps obtain the detection parameters from the environmental image more quickly, and determine whether the detection parameters meet detection conditions corresponding to the detection parameters. An image collected by the second camera is a face image, and is for detecting health status data. The image collection range of the second camera is narrowed down, to obtain a more accurate face area image to detect the health status data.

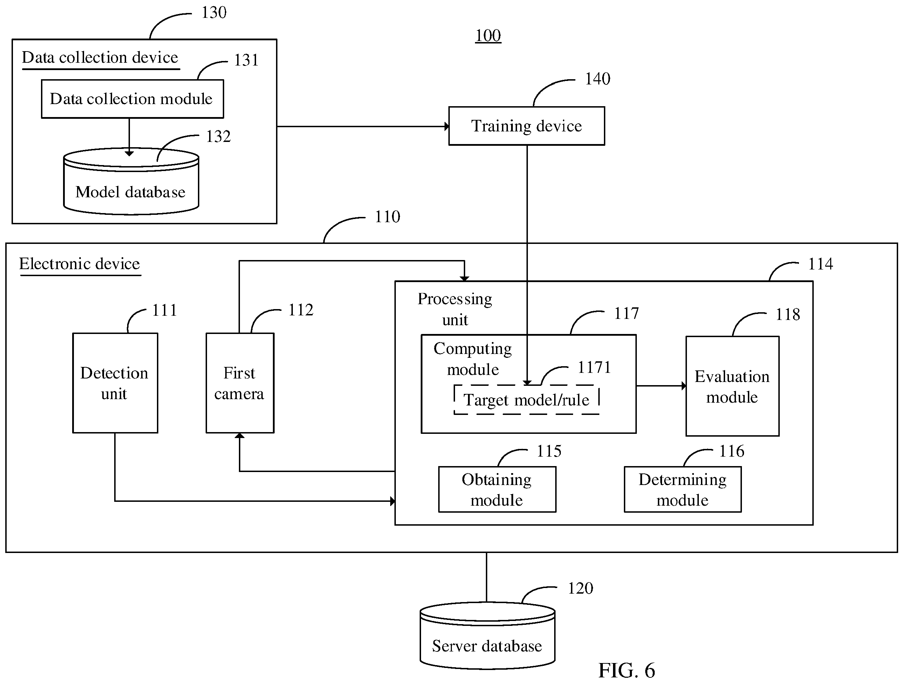

FIG. 6 is a schematic diagram of a structure of another system architecture according to an embodiment of the present invention. As shown in FIG. 6, a difference between the system architecture and the system architecture in FIG. 1 lies in that the system architecture in FIG. 1 includes the first camera 112 and the second camera 113, while the system architecture in FIG. 6 includes the first camera 112.

When the determining module 116 determines that the face image range is within the preset position range, the environmental luminance is greater than or equal to the first preset threshold, and the duration during which the face remains static is greater than or equal to the second preset threshold, the processing unit 114 invokes the first camera 112 over the health application, to control the first camera 112 to collect a face image.

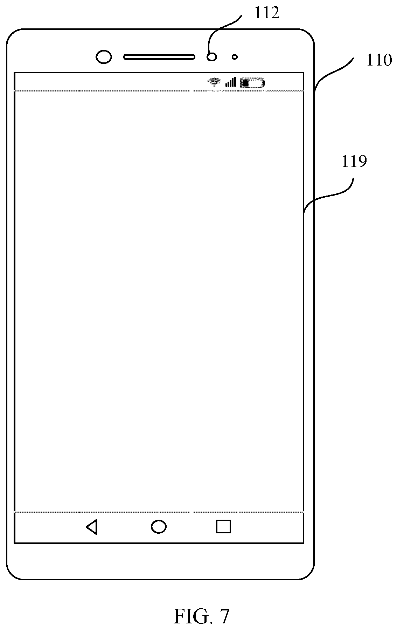

FIG. 7 is a schematic diagram of a structure of an electronic device having a display and a camera according to an embodiment of the present invention. The camera includes a first camera. As shown in FIG. 7, the electronic device 110 includes a display 119 and a first camera 112. The first camera 112 is located in a front panel area of the electronic device 110. The display 119 is configured to display a plurality of changing numbers, texts, graphic images, and the like. The first camera 112 may be a built-in camera, configured to photograph an image.

In this embodiment of the present invention, the first camera 112 is a low-power camera. The low-power camera is a camera capable of continuous photographing, and has advantages of low power consumption and capability of long-time operation.

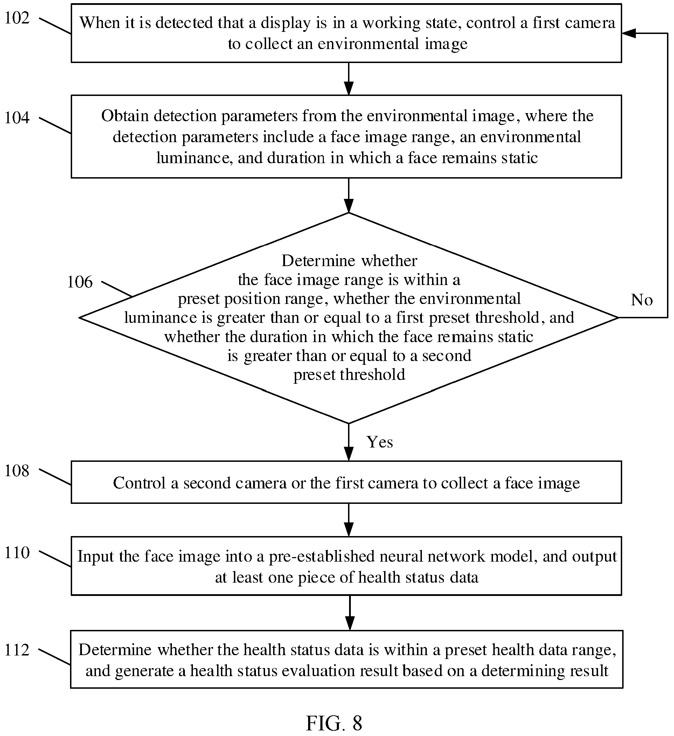

FIG. 8 is a flowchart of a health status detection method according to an embodiment of the present invention. As shown in FIG. 8, the method is applied to an electronic device having a display and a camera, and the camera includes a first camera or the camera includes a first camera and a second camera. The method includes the following steps.

Step 102: When it is detected that the display is in a working state, control the first camera to collect an environmental image.

In this embodiment of the present invention, as shown in FIG. 1 and FIG. 3, when the detection unit 111 detects that the display 119 is in a screen-on state, it indicates that the display 119 is in the working state. When the detection unit 111 detects that the display 119 is in a screen-off state, it indicates that the display 119 is in a non-working state.

When the detection unit 111 detects that the display 119 is in the working state, the detection unit 111 feeds back a detection result to the processing unit 114. The processing unit 114 invokes the first camera 112 based on the detection result over the health application, to control the first camera 112 to collect an environmental image 1. The first camera 112 is a low-power camera, and can continuously collect the environmental image 1.

The environmental image is an image collected by the first camera 112 in an environment in which the electronic device 110 is located, and the environmental image may include a face image.

In this embodiment of the present invention, before step 102, the method further includes the following steps.

Step 101a: Detect whether the health application is installed on the electronic device; and if yes, perform step 104; or if no, continue to perform step 101b.

The health application is an application for detecting a health status, and the health application is installed on the electronic device 110.

In this embodiment of the present invention, the processing unit 114 detects whether the health application is installed on the electronic device 110. If the processing unit 114 detects that the health application is installed on the electronic device 110, it indicates that the electronic device 110 meets a condition for performing health detection, and the processing unit 114 continues to perform step 102.

Step 101b: Install the health application, and continue to perform step 104.

In this embodiment of the present invention, when detecting that the health application is not installed on the electronic device 110, the processing unit 114 first installs the health application, and continues to perform step 104.

Step 104: Obtain detection parameters from the environmental image, where the detection parameters include a face image range, an environmental luminance, and duration in which a face remains static.

Step 104 in this embodiment may be performed by the obtaining module 115 in FIG. 1.

In this embodiment of the present invention, the step 104 specifically includes the following steps.

Step 104a: Obtain the face image range from the environmental image.

In this embodiment of the present invention, the obtaining the face image range from the environmental image specifically includes: identifying a face area from the environmental image; identifying a forehead area, a cheek area, and an organ area in the face area; and determining the face image range based on the forehead area, the cheek area, and the organ area.

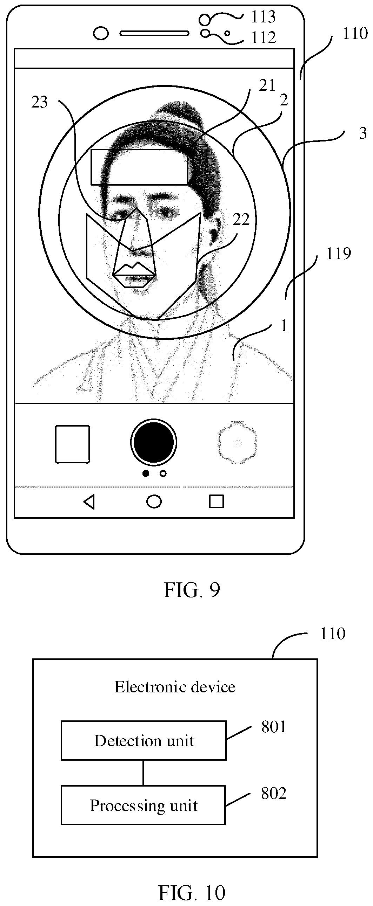

As shown in FIG. 1 and FIG. 9, after learning a facial feature through machine learning, the obtaining module 115 can identify a face area from the environment image 1, identify a forehead area 21, a cheek area 22, and an organ area 23 in the face area, and determine a face image range 2 based on the forehead area 21, the cheek area 22, and the organ area 23.

Step 104b: Obtain the environmental luminance from the environmental image.

In this embodiment of the present invention, the obtaining the environmental luminance from the environmental image specifically includes: identifying light source information from the environmental image; and identifying the luminance from the light source information. The light source information includes light sources such as natural light, incandescent light, and ambient light.

For example, the obtaining module 115 can identify, from the environmental image, that the light source information is natural light and a luminance of the natural light is 10 lux.

Step 104c: Obtain the duration in which the face remains static from the environmental image.

In this embodiment of the present invention, the obtaining the duration in which the face remains static from the environmental image specifically includes:

> obtaining positions of the forehead area, the cheek area, and the organ area in a face area in a current environmental image by starting from the first environmental image, where the first environmental image is the current environmental image;
>
> obtaining positions of the forehead area, the cheek area, and the organ area in a face area of a next environmental image;
>
> determining whether the positions of the forehead area, the cheek area, and the organ area in the face area obtained from the current environmental image are the same as the positions of the forehead area, the cheek area, and the organ area in the face area obtained from the next environmental image;
>
> if it is determined that the positions of the forehead area, the cheek area, and the organ area in the face area obtained from the current environmental image are the same as the positions of the forehead area, the cheek area, and the organ area in the face area obtained from the next environmental image, determining the next environmental image as a current environmental image, and continuing to perform the step of obtaining positions of the forehead area, the cheek area, and the organ area in a face area obtained from a next environmental image; and
>
> if it is determined that the positions of the forehead area, the cheek area, and the organ area in the face area obtained from the current environmental image are different from the positions of the forehead area, the cheek area, and the organ area in the face area obtained from the next environmental image, determining a time period between a collection time point of the first environmental image and a collection time point of a previous environmental image of the next environmental image as the duration in which the face remains static.

In this embodiment of the present invention, the first camera 112 may periodically collect the environmental image, for example, collect the environmental image every 0.1s. For example, when the first camera 112 collects the environmental image, the first camera 112 collects a total of 20 environmental images within 2s.

For example, if the obtaining module 115 determines that positions of the forehead area, the cheek area, and the organ area in a face area obtained from the first environmental image are the same as the positions of the forehead area, the cheek area, and the organ area in a face area obtained from the second environmental image, the obtaining module 115 continues to obtain positions of the forehead area, the cheek area, and the organ area in a face area obtained from the third environmental image. If it is determined that the positions of the forehead area, the cheek area, and the organ area in the face area obtained from the second environmental image are different from the positions of the forehead area, the cheek area, and the organ area in the face area obtained from the third environmental image, a time period between a collection time point of the first environmental image and a collection time point of the second environmental image is determined as the duration in which the face remains static.

Step 106: Determine whether the face image range is within a preset position range, whether the environmental luminance is greater than or equal to a first preset threshold, and whether the duration in which the face remains static is greater than or equal to a second preset threshold; and if yes, perform step 108; or if no, continue to perform step 102.

In this embodiment of the present invention, as shown in FIG. 1, if the determining module 116 determines that the face image range is within the preset position range, the environmental luminance is greater than or equal to the first preset threshold, and the duration in which the face remains static is greater than or equal to the second preset threshold, it indicates that the obtained detection parameters meet the detection conditions, and step 108 continues to be performed. If the determining module 116 determines that the face image range is not within the preset position range, the environmental luminance is less than the first preset threshold, or the duration in which the face remains static is less than the second preset threshold, it indicates that an obtained detection parameter does not meet a detection condition, and step 102 continues to be performed.

In this embodiment of the present invention, as shown in FIG. 9, the preset position range is a circular box 3 on the display 119. The circular box 3 may be a circular area that uses a central position of a face as a center of the circle and uses a specified distance as a radius. If the detection unit 111 determines that the face image range 2 is within the circular box 3, it indicates that the face image range 2 is within the preset position range, and the first camera 112 can collect a complete face image. Optionally, when the detection unit 111 determines that the face image range 2 at a specified range ratio is located in the circular box 3, it indicates that the face image is located in the preset position range, and the first camera 112 can collect a complete face image. The specified range ratio is greater than 80% and less than 100%, and in an optional method, the specified range ratio is 90%. This reduces difficulty in health status detection, and improves efficiency of health status detection.

In this embodiment of the present invention, the first preset threshold may be 10 lux. The first preset threshold may be set based on historical experience. When a luminance is greater than or equal to 10 lux, it indicates that a face image can be clearly obtained, so that information about subcutaneous blood flow can be obtained from the face image.

In this embodiment of the present invention, the second preset threshold may be 1s. In a process in which the first camera 112 collects an environmental image, the processing unit 114 may control the first camera 112 to collect an environmental image every 0.1s, or the processing unit 114 may control the first camera 112 to collect an environmental image every 0.5s. If the detection unit 111 determines that positions of the forehead area, the cheek area, and the organ area in the face obtained by the first camera 112 from environmental images collected within 1s do not change, it indicates that the face is in a static state currently, and duration in which the face remains static is 1s.

In this embodiment of the present invention, the detection condition is not limited to the three detection conditions listed above. In this embodiment of the present invention, the foregoing three detection parameters are obtained, and whether the foregoing three detection parameters meet the detection conditions corresponding to the detection parameters is determined, whether the electronic device 110 meets a condition for performing health detection can be determined. This improves health detection efficiency.

Step 108: Control the second camera or the first camera to collect a face image.

In this embodiment of the present invention, the second camera 113 includes an RGB camera. An RGB color mode is a color standard in the industry, in which channels of three colors: red (R), green (G), and blue (B) are changed and the three color channels are added to each other to obtain a broad array of colors. RGB indicates colors of red, green, and blue channels. This standard includes almost all colors that can be perceived by human vision, and is one of the most widely used color systems at present. Therefore, an image collected by the RGB camera is better than an image collected by the low-power camera in color and definition. Because the first camera 112 is a low-power camera, photographing quality is relatively low. Therefore, the second camera 113 is used to collect a face image, so that an obtained face image has a higher color and definition. This improves accuracy of face image analysis and improves efficiency of health status detection.

Step 110: Input the face image into a pre-established neural network model, and output at least one piece of health status data.

In this embodiment of the present invention, the at least one piece of health status data includes a skin status, a heart rate, blood pressure, or body fat. In a solution, one piece of health status data may include at least one of a status, a heart rate, blood pressure, and body fat. The calculation module 117 may output the four types of health status data based on a same face image input by the first camera 112 or the second camera 113, but time for outputting the four types of health status data is different. For example, in the calculation module 117, health status data of the heart rate and the blood pressure is obtained by a large amount of calculation performed by the target model/rule 1171. Therefore, it takes a long time to output the health status data of the heart rate and the blood pressure.

In this embodiment of the present invention, for example, as shown in FIG. 1, after the processing unit 114 invokes the second camera 113 over the health application to control the second camera 113 to collect the face image, the face image is input into the target model/rule 1171, and the at least one piece of health status data is output. After the face image is input into the target model/rule 1171, in the target model/rule 1171, after the obtained face image is converted into face image parameters, features of a skin area that are extracted by a convolutional layer undergo processing of a pooling layer and digital processing of a fully connected layer to generate skin area parameters, so as to identify the skin area. Based on the different skin area parameters, a subcutaneous blood flow image feature of the skin area is determined. Further, a correspondence between the subcutaneous blood flow image feature and the health status data is established in the target model/rule 1171 of the calculation module 117, so that corresponding health status data is output based on the subcutaneous blood flow image feature.

For example, the subcutaneous blood flow image feature includes a feature of a color change frequency of a subcutaneous blood vessel. A correspondence between a feature of a subcutaneous blood color change frequency and heart rate data is established in the target model/rule 1171. To be specific, each time a color of a subcutaneous blood vessel changes, corresponding heart rate data can be obtained based on the correspondence between the feature of the subcutaneous blood color change frequency and the heart rate data. The heart rate data is output from the target model/rule 1171 based on consecutive features of the subcutaneous blood color change frequency in frequency domain. For example, face images in a preset time period are obtained. After the face images in the preset time period are input into the target model/rule 1171, a subcutaneous blood color feature in the preset time period is obtained from the target model/model 1171, and corresponding heart rate data is output from the target model/rule 1171 based on a feature of a subcutaneous blood color change frequency in frequency domain in the preset time period. The heart rate data is not a fixed value, but is dynamic heart rate data output based on the feature of the subcutaneous blood color change frequency in frequency domain in the preset time period. For example, the preset time period is 10s.

Step 112: Determine whether the health status data is within a preset health data range, and generate a health status evaluation result based on a determining result.

In this embodiment of the present invention, for example, a value range of a heart rate health index in the preset health data range includes 65 to 70, but the health status data of the heart rate obtained in step 112 is 75. Therefore, the evaluation module 118 determines that the value of the health status data of the heart rate is high, and provides, based on a determining result indicating that the heart rate is high, a health status evaluation result corresponding to the high heart rate. The health status evaluation result includes a health status evaluation conclusion and health status guidance information corresponding to the health status evaluation conclusion. For example, the health status evaluation conclusion includes a high heart rate. The health status guidance information includes information such as eating frequent small meals, avoiding overeating, and eating low-calorie foods.

In this embodiment of the present invention, the method further includes the following step:
Step 114: Store the at least one piece of health status data to a local database, and generate a detection report based on the at least one piece of health status data.

In this embodiment of the present invention, the processing unit 114 generates the detection report based on the at least one piece of health status data, so that a user can learn health status data of the user in time.

In this embodiment of the present invention, after step 114, optionally, the method further includes the following step:
Step 114': Send the at least one piece of health status data to a cloud server, so that the cloud server stores the at least one piece of health status data, generates a detection report based on the at least one piece of health status data, and returns the detection report to the electronic device.

In this embodiment of the present invention, the health status data is stored in the cloud server. In this way, after replacing the electronic device 110, the user can still query historical health status data and a historical detection report on the cloud server.

In the technical solution provided in this embodiment of the present invention, the first camera is controlled to collect the environmental image, and the detection parameters are obtained from the environmental image. If it is determined that the detection parameters meet the detection condition corresponding to the detection parameters, the second camera or the first camera is controlled to collect the face image, and health status data is generated based on the face image. In this embodiment of the present invention, the first camera collects the environmental image to determine the detection conditions corresponding to the detection parameters, and the second camera or the first camera is controlled to collect the face image to generate the health status data. A user does not need to manually perform health status detection, but health status detection can be automatically performed, to implement senseless health status detection.

FIG. 10 is a schematic diagram of a structure of an electronic device according to an embodiment of the present invention. It should be understood that an electronic device 110 can perform the steps in the foregoing health status detection method. To avoid repetition, details are not described herein again. The electronic device 110 includes a detection unit 801 and a processing unit 802.

The detection unit 801 is configured to detect whether a display is in a working state. The processing unit 802 is configured to: when the detection unit 801 detects that the display is in the working state, control the first camera to collect an environmental image. The processing unit 802 is further configured to obtain detection parameters from the environmental image. The processing unit 802 is further configured to determine whether the detection parameters meet detection conditions corresponding to the detection parameter; and if the detection unit determines that the detection parameters meet the detection conditions corresponding to the detection parameters, control the second camera or the first camera to collect a face image. The processing unit 802 is further configured to generate at least one piece of health status data based on the face image.

In a possible implementation, the processing unit 802 is configured to input the face image into a pre-established neural network model, and output the at least one piece of health status data.

In a possible implementation, the at least one piece of health status data includes at least one of a skin status, a heart rate, blood pressure, body fat.

In a possible implementation, the detection parameters include an environmental luminance, a face image range, and duration in which a face remains static.

In a possible implementation, the processing unit 802 is configured to determine whether the face image range is within a preset position range, whether the environmental luminance is greater than or equal to a first preset threshold, and whether the duration in which the face remains static is greater than or equal to a second preset threshold.

In a possible implementation, the processing unit 802 is configured to determine whether the health status data is within a preset health data range, and generate a health status evaluation result based on a determining result.

In a possible implementation, the first camera includes a low-power camera.

It should be understood that the information display device 110 herein is presented in a form of a functional unit. The term "unit" herein may be implemented in a form of software and/or hardware. This is not specifically limited. For example, the "unit" may be a software program, a hardware circuit, or a combination thereof for implementing the foregoing function. The hardware circuit may include an application-specific integrated circuit (application-specific integrated circuit, ASIC), an electronic circuit, a processor (for example, a shared processor, a dedicated processor, or a group processor) configured to execute one or more software or firmware programs and a memory, a merged logic circuit, and/or another appropriate component that supports the described function.

Therefore, the units in the examples described in embodiments of the present invention can be implemented by using electronic hardware, or a combination of computer software and electronic hardware. Whether the functions are performed by hardware or software depends on particular applications and design constraints of the technical solutions. A person skilled in the art may use different methods to implement the described functions for each particular application, but it should not be considered that the implementation goes beyond the scope of the present invention.

An embodiment of the present invention further provides an electronic device. The electronic device may be a terminal device, or may be a circuit device built into the terminal device. The device may be configured to perform the functions/steps in the foregoing method embodiments.

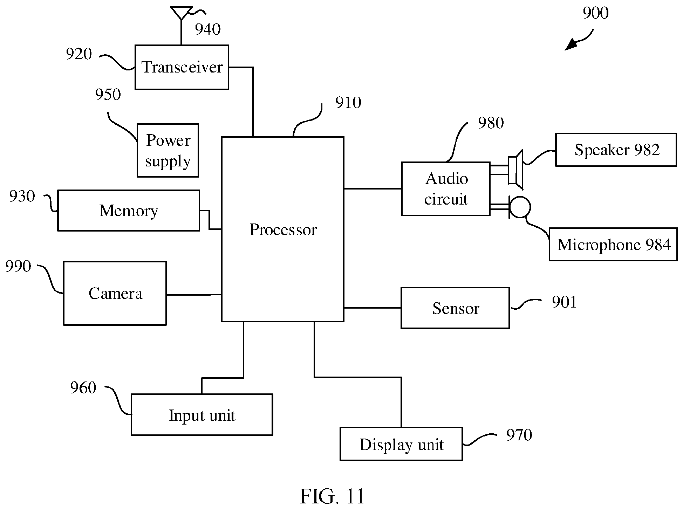

FIG. 11 is a schematic diagram of a structure of another electronic device according to an embodiment of the present invention. As shown in FIG. 11, an electronic device 900 includes a processor 910 and a transceiver 920. Optionally, the electronic device 900 may further include a memory 930. The processor 910, the transceiver 920, and the memory 930 may communicate with each other through an internal connection path to transfer a control signal and/or a data signal. The memory 930 is configured to store a computer program. The processor 910 is configured to: invoke and run the computer program in the memory 930.

Optionally, the electronic device 900 may further include an antenna 940, configured to send a wireless signal output by the transceiver 920.

The processor 910 and the memory 930 may be integrated into one processing apparatus, or more commonly be components independent of each other. The processor 910 is configured to execute program code stored in the memory 930 to implement the foregoing functions. In a specific implementation, the memory 930 may also be integrated into the processor 910, or may be independent of the processor 910. The processor 910 may correspond to the processing unit 802 in the electronic device 900 in FIG. 10.

In addition, the electronic device 900 may further include one or more of an input unit 960, a display unit 970, an audio circuit 980, a camera 990, a sensor 901, and the like, to improve the functions of the electronic device 900. The audio circuit may further include a speaker 982, a microphone 984, and the like. The display unit 970 may include a display.

Optionally, the electronic device 900 may further include a power supply 950, configured to supply power to various components or circuits in the terminal device.

It should be understood that the electronic device 900 shown in FIG. 11 can implement processes of the embodiment of the health status detection method shown in FIG. 8. Operations and/or functions of the modules of the electronic device 900 are separately intended to implement a corresponding process in the foregoing method embodiments. For details, refer to the descriptions in the foregoing method embodiments. To avoid repetition, detailed descriptions are appropriately omitted herein.

It should be understood that the processor 910 in the electronic device 900 shown in FIG. 11 may be a system on a chip (system on a chip, SOC). The processor 910 may include a central processing unit (central processing unit, CPU), and may further include another type of processor. The CPU may be referred to as a host CPU. A neural network processing unit NPU 30 is mounted to the host CPU (Host CPU) as a coprocessor, and the host CPU assigns a task. The processors work together to implement the foregoing method procedures, and each processor may selectively execute a part of software drivers.

For example, steps 102 to 114 in FIG. 8 may be performed by the CPU, or may be performed by a DSP.

In conclusion, some processors or processing units in the processor 910 may operate together to implement the procedure of the foregoing method, and software programs corresponding to the processors or processing units may be stored in the memory 930. The NPU 30 is merely used as an example. An actual neural network function may be performed by a processing device other than the NPU 30. For example, a graphics processing unit (graphics processing unit, GPU) may also be used for neural network processing. This is not limited in this embodiment.

The present invention further provides a computer storage medium. The computer storage medium stores instructions. When the instructions are run on a computer, the computer is enabled to perform the steps in the health status detection method shown in FIG. 8.

In the foregoing embodiments, the processor 910 may include, for example, a central processing unit (central processing unit, CPU), a microprocessor, a microcontroller, or a digital signal processor, and may further include a GPU, an NPU, and an ISP. The processor may further include a necessary hardware accelerator or a logic processing hardware circuit, for example, an application-specific integrated circuit (application-specific integrated circuit, ASIC), or one or more integrated circuits configured to control programs to perform the technical solutions in the present invention. In addition, the processor may have a function of operating one or more software programs, and the software program may be stored in the memory.

The memory may be a read-only memory (read-only memory, ROM), another type of static storage device that can store static information and instructions, or a random access memory (random access memory, RAM) or another type of dynamic storage device that can store information and instructions, or may be an electrically erasable programmable read-only memory (electrically erasable programmable read-only memory, EEPROM), a compact disc read-only memory (compact disc read-only memory, CD-ROM) or another compact disc storage medium, an optical disc storage medium (including a compact optical disc, a laser disc, an optical disc, a digital versatile optical disc, a Blu-ray disc, and the like), a magnetic disk storage medium or another magnetic storage device, any other medium that can be used to carry or store expected program code in a form of instructions or a data structure and that can be accessed by a computer, or the like.

In embodiments of the present invention, "at least one" refers to one or more, and "a plurality of" refers to two or more. The term "and/or" describes an association relationship for describing associated objects and indicates that three relationships may exist. For example, A and/or B may indicate the following three cases: Only A exists, both A and B exist, and only B exists. A and B may be in a singular form or a plural form. A character "/" generally indicates an "or" relationship between the associated objects. At least one of the following items and similar expressions refer to any combination of the items, including a single item or any combination of plural items. For example, at least one of a, b, and c may indicate a, b, c, a and b, a and c, b and c, or a, b, and c, where a, b, and c may be singular or plural.

A person of ordinary skill in the art may be aware that, with reference to the examples described in embodiments disclosed in this specification, units and algorithm steps may be implemented by electronic hardware or a combination of computer software and electronic hardware. Whether the functions are performed by hardware or software depends on particular applications and design constraints of the technical solutions. A person skilled in the art may use different methods to implement the described functions for each particular application, but it should not be considered that the implementation goes beyond the scope of the present invention.

It may be clearly understood by a person skilled in the art that, for the purpose of convenient and brief description, for a detailed working process of the foregoing system, apparatus, and unit, refer to a corresponding process in the foregoing method embodiments. Details are not described herein again.

In embodiments of the present invention, when any of the functions are implemented in a form of a software functional unit and sold or used as an independent product, the functions may be stored in a computer storage medium. Based on such an understanding, the technical solutions of the present invention essentially, or the part contributing to the prior art, or some of the technical solutions may be implemented in a form of a software product. The computer software product is stored in a storage medium, and includes several instructions for instructing a computer device (which may be a personal computer, a server, or a network device) to perform all or some of the steps of the methods described in embodiments of the present invention. The foregoing storage medium includes: any medium that can store program code, such as a USB flash drive, a removable hard disk, a read-only memory (read-only memory, ROM), a random access memory (random access memory, RAM), a magnetic disk, or a compact disc.

The above descriptions are merely specific implementations of the present invention. Any variation or replacement readily figured out by a person skilled in the art within the technical scope disclosed in the present invention shall fall within the protection scope of the present invention. The protection scope of the present invention shall be subject to the protection scope of the claims.

**Claims**

1. A health status detection method, wherein the method is applied to an electronic device having a display and a camera, and the camera comprises a first camera and a second camera, wherein the first camera is a low-power camera configured to acquire continuous photographing, and wherein the second camera is configured to acquire an image with a better color and definition than an image acquired by the first camera; and the method comprises:

> when it is detected that the display is in a working state, controlling (102) the first camera to collect an environmental image;
> obtaining (104) detection parameters from the environmental image, wherein the detection parameters comprise an environmental luminance, a face image range, and duration in which a face remains static;
> determining (106) whether the detection parameters meet detection conditions corresponding to the detection parameters, comprising determining whether the face image range is within a preset position range, whether the environmental luminance is greater than or equal to a first preset threshold, and whether the duration in which the face remains static is greater than or equal to a second preset threshold;
> if it is determined that the detection parameters meet the detection conditions corresponding to the detection parameters, controlling (108) the second camera to collect a face image; and
> generating (110) at least one piece of health status data based on the face image.

2. The method according to claim 1, wherein the generating at least one piece of health status data based on the face image comprises:
inputting the face image into a pre-established neural network model, and outputting the at least one piece of health status data.

3. The method according to claim 1, wherein the health status data comprises at least one of a skin status, a heart rate, blood pressure, and body fat.

4. The method according to claim 1, wherein after the generating health status data based on the face image, the method further comprises:
determining whether the health status data is within a preset health data range, and generating a health status evaluation result based on a determining result.

5. A health status detection device (110, 900), comprising:

> a display (119); a camera, wherein the camera comprises a first camera (112) and a second camera (113), wherein the first camera is a low-power camera configured to acquire continuous photographing, and wherein the second camera is configured to acquire an image with a better color and definition than an image acquired by the first camera; one or more processors (910); a memory (930); a plurality of applications; and one or more computer programs, wherein the one or more computer programs are stored in the memory, the one or more computer programs comprise instructions, and when the instructions are executed by the device, the device is enabled to perform the following steps:
> when it is detected that the display is in a working state, controlling the first camera to collect an environmental image;
> obtaining detection parameters from the environmental image, wherein the detection parameters comprise an environmental luminance, a face image range, and duration in which a face remains static;
> determining whether the detection parameters meet detection conditions corresponding to the detection parameters, comprising determining whether the face image range is within a preset position range, whether the environmental luminance is greater than or equal to a first preset threshold, and whether the duration in which the face remains static is greater than or equal to a second preset threshold;
> if it is determined that the detection parameters meet the detection conditions corresponding to the detection parameters, controlling the second camera to collect a face image; and
> generating at least one piece of health status data based on the face image.

6. The device according to claim 5, wherein when the instructions are executed by the device, the device is enabled to specifically perform the following steps:
inputting the face image into a pre-established neural network model, and outputting the at least one piece of health status data.

7. The device according to claim 5, wherein the at least one piece of health status data comprises a skin status, a heart rate, blood pressure, and body fat.

8. The device according to claim 5, wherein when the instructions are executed by the device, the device is enabled to specifically perform the following steps:
determining whether the health status data is within a preset health data range, and generating a health status evaluation result based on a determining result.

9. A computer storage medium, comprising computer instructions, wherein when the computer instructions are run on the health status detection device of any one of claims 5-8, the electronic device is enabled to perform the health status detection method according to any one of claims 1 to 4.

**Patentansprüche**

1. Verfahren zur Erkennung des Gesundheitszustands, wobei das Verfahren auf eine elektronische Vorrichtung mit einer Anzeige und einer Kamera angewendet wird und die Kamera eine erste Kamera und eine zweite Kamera umfasst, wobei die erste Kamera eine Niedrigenergiekamera ist, die zum kontinuierlichen fotografischen Aufnehmen konfiguriert ist, und wobei die zweite Kamera dazu konfiguriert ist, ein Bild mit besserer Farbe und Auflösung als ein von der ersten Kamera aufgenommenes Bild aufzunehmen; und das Verfahren umfasst:

   wenn erkannt wird, dass sich die Anzeige in einem Arbeitszustand befindet, Steuern (102) der ersten Kamera, um ein Umgebungsbild zu erfassen;
   Erlangen (104) von Erkennungsparametern aus dem Umgebungsbild, wobei die Erkennungsparameter eine Umgebungsleuchtdichte, einen Gesichtsbildbereich und eine Dauer umfassen, in der ein Gesicht statisch verbleibt;
   Bestimmen (106), ob die Erkennungsparameter den Erkennungsparametern entsprechende Erkennungsbedingungen erfüllen, umfassend das Bestimmen, ob der Gesichtsbildbereich innerhalb eines voreingestellten Positionsbereichs liegt, ob die Umgebungsleuchtdichte größer oder gleich einem ersten voreingestellten Schwellenwert ist, und ob die Dauer, in der das Gesicht statisch verbleibt, größer oder gleich einem zweiten voreingestellten Schwellenwert ist;
   wenn bestimmt wird, dass die Erkennungsparameter die den Erkennungsparametern entsprechenden Erkennungsbedingungen erfüllen, Steuern (108) der zweiten Kamera, um ein Gesichtsbild zu erfassen; und
   Erzeugen (110) von mindestens einem Gesundheitszustandsdatum basierend auf dem Gesichtsbild.

2. Verfahren nach Anspruch 1, wobei das Erzeugen von mindestens einem Gesundheitszustandsdatum basierend auf dem Gesichtsbild umfasst:
   Eingeben des Gesichtsbildes in ein voreingerichtetes neuronales Netzwerkmodell und Ausgeben des mindestens einen Gesundheitszustandsdatums.

3. Verfahren nach Anspruch 1, wobei die Gesundheitszustandsdaten mindestens eines von Hautzustand, Herzfrequenz, Blutdruck und Körperfett umfassen.

4. Verfahren nach Anspruch 1, wobei das Verfahren nach dem Erzeugen von Gesundheitszustandsdaten basierend auf dem Gesichtsbild ferner umfasst:
   Bestimmen, ob die Gesundheitszustandsdaten innerhalb eines voreingestellten Gesundheitsdatenbereichs liegen, und Erzeugen eines Gesundheitszustands-Bewertungsergebnisses basierend auf einem Bestimmungsergebnis.

5. Vorrichtung zur Erkennung des Gesundheitszustands (110, 900), umfassend:
   eine Anzeige (119); eine Kamera, wobei die Kamera eine erste Kamera (112) und eine zweite Kamera (113) umfasst, wobei die erste Kamera eine Niedrigenergiekamera ist, die zum kontinuierlichen fotografischen Aufnehmen konfiguriert ist, und wobei die zweite Kamera dazu konfiguriert ist, ein Bild mit besserer Farbe und Auflösung als ein von der ersten Kamera aufgenommenes Bild aufzunehmen; einen oder mehrere Prozessoren (910); einen Speicher (930); eine Vielzahl von Anwendungen; und ein oder mehrere Computerprogramme, wobei das eine oder die mehreren Computerprogramme in dem Speicher gespeichert sind, das eine oder die mehreren Computerprogramme Anweisungen umfassen, und wenn die Anweisungen durch die Vorrichtung ausgeführt werden, die Vorrichtung befähigt wird, die folgenden Schritte durchzuführen:

   wenn erkannt wird, dass sich die Anzeige in einem Arbeitszustand befindet, Steuern der ersten Kamera, um ein Umgebungsbild zu erfassen;
   Erlangen von Erkennungsparametern aus dem Umgebungsbild, wobei die Erkennungsparameter eine Umgebungsleuchtdichte, einen Gesichtsbildbereich und eine Dauer umfassen, in der ein Gesicht statisch verbleibt;

Bestimmen, ob die Erkennungsparameter den Erkennungsparametern entsprechende Erkennungsbedingungen erfüllen, umfassend das Bestimmen, ob der Gesichtsbildbereich innerhalb eines voreingestellten Positionsbereichs liegt, ob die Umgebungsleuchtdichte größer oder gleich einem ersten voreingestellten Schwellenwert ist, und ob die Dauer, in der das Gesicht statisch verbleibt, größer oder gleich einem zweiten voreingestellten Schwellenwert ist;

wenn bestimmt wird, dass die Erkennungsparameter die den Erkennungsparametern entsprechenden Erkennungsbedingungen erfüllen, Steuern der zweiten Kamera, um ein Gesichtsbild zu erfassen; und

Erzeugen von mindestens einem Gesundheitszustandsdatum basierend auf dem Gesichtsbild.

6. Vorrichtung nach Anspruch 5, wobei, wenn die Anweisungen durch die Vorrichtung ausgeführt werden, die Vorrichtung befähigt wird, speziell die folgenden Schritte durchzuführen:

Eingeben des Gesichtsbildes in ein voreingerichtetes neuronales Netzwerkmodell und Ausgeben des mindestens einen Gesundheitszustandsdatums.

7. Vorrichtung nach Anspruch 5, wobei das mindestens eine Gesundheitszustandsdatum einen Hautzustand, eine Herzfrequenz, Blutdruck und Körperfett umfasst.

8. Vorrichtung nach Anspruch 5, wobei, wenn die Anweisungen durch die Vorrichtung ausgeführt werden, die Vorrichtung befähigt wird, speziell die folgenden Schritte durchzuführen:

Bestimmen, ob die Gesundheitszustandsdaten innerhalb eines voreingestellten Gesundheitsdatenbereichs liegen, und Erzeugen eines Gesundheitszustands-Bewertungsergebnisses basierend auf einem Bestimmungsergebnis.

9. Computerspeichermedium, umfassend Computeranweisungen, wobei, wenn die Computeranweisungen auf der Vorrichtung zur Erkennung des Gesundheitszustands nach einem der Ansprüche 5-8 ausgeführt werden, die elektronische Vorrichtung befähigt wird, das Verfahren zur Erkennung des Gesundheitszustands nach einem der Ansprüche 1 bis 4 durchzuführen.

**Revendications**

1. Procédé de détection d'état de santé, dans lequel le procédé est appliqué à un dispositif électronique ayant un écran et une caméra, et la caméra comprend une première caméra et une seconde caméra, dans lequel la première caméra est une caméra basse consommation configurée pour acquérir des photos en continu, et dans lequel la seconde caméra est configurée pour acquérir une image avec une meilleure couleur et une meilleure définition qu'une image acquise par la première caméra ; et le procédé comprend :

lorsqu'il est détecté que l'écran est dans un état de fonctionnement, la commande (102) de la première caméra pour collecter une image environnementale ;

l'obtention (104) de paramètres de détection à partir de l'image environnementale, dans lequel les paramètres de détection comprennent une luminance environnementale, une plage d'images de visage, et une durée pendant laquelle un visage reste statique ; le fait de déterminer (106) si les paramètres de détection satisfont les conditions de détection correspondant aux paramètres de détection, comprenant le fait de déterminer si la plage d'images de visage est dans une plage de positions prédéfinie, si la luminance environnementale est supérieure ou égale à un premier seuil prédéfini, et si la durée pendant laquelle le visage reste statique est supérieure ou égale à un second seuil prédéfini ;

s'il est déterminé que les paramètres de détection répondent aux conditions de détection correspondant aux paramètres de détection, la commande (108) de la seconde caméra pour collecter une image de visage ; et

la génération (110) d'au moins un élément de données d'état de santé sur la base de l'image de visage.

2. Procédé selon la revendication 1, dans lequel la génération d'au moins un élément de données d'état de santé sur la base de l'image de visage comprend :

la saisie de l'image de visage dans un modèle de réseau neuronal préétabli et l'émission de l'au moins un élément de données d'état de santé.

3. Procédé selon la revendication 1, dans lequel les données d'état de santé comprennent au moins l'un d'un état de peau, d'une fréquence cardiaque, d'une pression artérielle, et d'une masse grasse corporelle.

4. Procédé selon la revendication 1, dans lequel après la génération de données d'état de santé sur la base de l'image de visage, le procédé comprend également :
le fait de déterminer si les données d'état de santé sont dans une plage de données de santé prédéfinie, et la génération d'un résultat d'évaluation d'état de santé sur la base d'un résultat déterminant.

5. Dispositif de détection d'état de santé (110, 900), comprenant : un écran (119) ; une caméra, dans lequel la caméra comprend une première caméra (112) et une seconde caméra (113), dans lequel la première caméra est une caméra basse consommation configurée acquérir des photos en continu, et dans lequel la seconde caméra est configurée pour acquérir une image avec une meilleure couleur et une meilleure définition qu'une image acquise par la première caméra ; un ou plusieurs processeurs (910) ; une mémoire (930) ; une pluralité d'applications ; et un ou plusieurs programmes informatiques, dans lequel les un ou plusieurs programmes informatiques sont stockés dans la mémoire, les un ou plusieurs programmes informatiques comprennent des instructions, et lorsque les instructions sont exécutées par le dispositif, le dispositif est autorisé à réaliser les étapes suivantes :

lorsqu'il est détecté que l'écran est dans un état de fonctionnement, la commande de la première caméra pour collecter une image environnementale ;
l'obtention de paramètres de détection à partir de l'image environnementale, dans lequel les paramètres de détection comprennent une luminance environnementale, une plage d'images de visage, et une durée pendant laquelle un visage reste statique ; le fait de déterminer si les paramètres de détection satisfont les conditions de détection correspondant aux paramètres de détection, comprenant le fait de déterminer si la plage d'images de visage est dans une plage de positions prédéfinie, si la luminance environnementale est supérieure ou égale à un premier seuil prédéfini, et si la durée pendant laquelle le visage reste statique est supérieure ou égale à un second seuil prédéfini ;
s'il est déterminé que les paramètres de détection répondent aux conditions de détection correspondant aux paramètres de détection, la commande de la seconde caméra pour collecter une image de visage ; et
la génération d'au moins un élément de données d'état de santé sur la base de l'image de visage.

6. Dispositif selon la revendication 5, dans lequel lorsque les instructions sont exécutées par le dispositif, le dispositif est autorisé à réaliser spécifiquement les étapes suivantes :
la saisie de l'image de visage dans un modèle de réseau neuronal préétabli et l'émission de l'au moins un élément de données d'état de santé.

7. Procédé selon la revendication 5, dans lequel l'au moins un élément de données d'état de santé comprend un état de peau, une fréquence cardiaque, une pression artérielle, et une masse grasse corporelle.

8. Dispositif selon la revendication 5, dans lequel lorsque les instructions sont exécutées par le dispositif, le dispositif est autorisé à réaliser spécifiquement les étapes suivantes :
le fait de déterminer si les données d'état de santé sont dans une plage de données de santé prédéfinie, et la génération d'un résultat d'évaluation d'état de santé sur la base d'un résultat déterminant.

9. Support de stockage informatique, comprenant des instructions informatiques, dans lequel, lorsque les instructions informatiques fonctionnent sur le dispositif de détection d'état de santé selon l'une quelconque des revendications 5 à 8, le dispositif électronique est autorisé à réaliser le procédé de détection d'état de santé selon l'une quelconque des revendications 1 à 4.

FIG. 1

Neural network layer 230

Output layer 240

Hidden layer n (23n)

Hidden layer 2 (232)

Hidden layer 1 (231)

Convolution layer/pooling layer 220

226

225

224

223

222

221

Convolutional neural network (CNN) 200

Input layer 210

Face image

FIG. 2

FIG. 3

FIG. 4

FIG. 5

FIG. 6

FIG. 7

FIG. 8

FIG. 9

FIG. 10

FIG. 11

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 109363634 A

- CN 108509905 A